# FASCICULE DE BREVET EUROPEEN

(11) **EP 2 682 095 B1**
(45) Date de publication et mention de la délivrance du brevet: **13.05.2015**
(21) Numéro de dépôt: 13305935.2
(22) Date de dépôt: 01.07.2013
(51) Int. Cl.: A61K 8/49, A61Q 19/08, C07C 237/08, C07D 209/14, C07D 233/64, A61P 17/00, A61K 31/417, A61K 31/4045, A61K 31/165

(54) **Famille de composés aryléthylamides polyaminés, et leurs applications cosmétiques ou dermocosmétiques**
Familie von Polyamin-Arylethylamiden, und ihre kosmetische oder hautkosmetische Anwendung
Family of polyamine arylethylamide compounds, and their cosmetic or dermocosmetic use

(30) Priorité: 02.07.2012 FR 1256310
(43) Date de publication de la demande: 08.01.2014
(73) Titulaire: Exsymol, 98000 Monaco (MC)
(72) Inventeur: Seguin, Marie-Christine, 98000 MONACO (MC)
(74) Mandataire: Nevant, Marc

(56) Documents cités:
- WO-A1-95/12581
- WO-A2-02/062755
- FR-A1- 2 833 165
- PEIYU SUN ET AL: "Facile and universal immobilization of l-lysine inspired by mussels", JOURNAL OF MATERIALS CHEMISTRY, vol. 22, no. 19, 1 janvier 2012 (2012-01-01), page 10035, XP055054143, ISSN: 0959-9428, DOI: 10.1039/c2jm16598h
- RONG ZHU ET AL: "Functionalization of magnetic nanoparticles with peptide dendrimers", JOURNAL OF MATERIALS CHEMISTRY, vol. 21, no. 14, 1 janvier 2011 (2011-01-01), page 5464, XP055054144, ISSN: 0959-9428, DOI: 10.1039/c0jm02752a
- CLAUDIU T. SUPURAN ET AL: "Carbonic anhydrase activators: amino acyl/dipeptidyl histamine derivatives bind with high affinity to isozymes I, II and IV and act as efficient activators", BIOORGANIC & MEDICINAL CHEMISTRY, vol. 7, no. 12, 1 décembre 1999 (1999-12-01), pages 2915-2923, XP055054184, ISSN: 0968-0896, DOI: 10.1016/S0968-0896(99)00227-8
- L. S. KOROLEVA ET AL: "Artificial ribonucleases: Quantitative analysis of the structure-activity relationship and a new insight into the strategy of design of highly efficient RNase mimetics", RUSSIAN JOURNAL OF BIOORGANIC CHEMISTRY, vol. 34, no. 4, 1 juillet 2008 (2008-07-01), pages 442-452, XP055054182, ISSN: 1068-1620, DOI: 10.1134/S1068162008040080

## Description

L'invention a pour objet une famille de composés stables aryléthylamides polyaminés, et l'utilisation de ces composés comme agents inhibiteurs des dommages de l'ADN induits par les sous-produits de la glycosylation non enzymatique des tissus cutanés.

L'invention concerne également des compositions cosmétiques ou dermocosmétiques destinées à lutter contre les désordres cutanés associés à ces mêmes sous-produits de glycosylation.

Le processus de glycosylation non enzymatique des protéines est un phénomène identifié de longue date, consistant initialement en la condensation spontanée de sucres réducteurs, tels le glucose ou le fructose, avec les fonctions aminées N-terminales de certains constituants protéiques ou lipoprotéiques, tels les aminoacides lysine et arginine. A la différence du processus de glycosylation enzymatique, génétiquement programmé, un tel processus conduit défavorablement, après une suite de réactions en chaîne et des réarrangements moléculaires complexes, à des altérations irréversibles des protéines.

La nuisance de ces altérations sur les tissus biologiques, et les cellules qui les composent, est clairement établie, avec des conséquences physiologiques multiples : altérations fonctionnelles des protéines conduisant à des dysfonctionnements du métabolisme cellulaire (perturbations des activités enzymatiques), altérations des propriétés mécaniques de certains tissus de soutien, déclenchement de processus inflammatoires ou de stress oxydatif avec la production de cytokines ou d'espèces réactives dérivées de l'oxygène, atteintes au processus de réparation, etc. Il est d'ailleurs démontré que de telles modifications des protéines jouent un rôle essentiel dans le développement ou l'accélération de certaines pathologies, notamment liées à l'âge, telles le diabète sucré, l'athérosclérose, les maladies d'Alzheimer et de Parkinson, l'insuffisance rénale, etc (J. Uribarri et al., J. Gerontol. A Biol. Sci. Med. sci. (2007), vol.62, pp.437-433).

Concernant la peau de manière spécifique, l'impact du processus de glycosylation non enzymatique des protéines, en particulier sur son vieillissement, est sans conteste. En effet, une fois glyquées par la condensation spontanée du sucre, les protéines cutanées, en particulier celles de structure telle que le collagène ou l'élastine, se rigidifient avec l'accumulation de liaisons croisées covalentes interfibres. Les propriétés visco-élastiques de la peau se trouvent être alors amoindries, accentuant les rides naissantes ou formées. Le renouvellement de ces protéines cutanées est également ralenti (Dyer D.G. et al., J. Clin. Invest. (1993), vol.91, pp.2463-9).

Une autre connaissance bien acquise aujourd'hui, et non des moindres, sur le processus de glycosylation non enzymatique des protéines est que ce processus complexe libère aussi des substances, que l'on désigne souvent par le terme de "sous-produits de glycosylation", qui se comportent comme de véritables toxines. D'ailleurs, à l'instar d'un récent article et de son titre « Glycotoxins : a possible threat to health ? » (Odetti P. et al., Mediterr. J. Nutr. Metab., (2008), vol.l, pp. 63-67), il est de plus en plus relevé dans la littérature, et tel l'exposé de l'invention ci-après, la terminologie plus suggestive de "glycotoxines" pour désigner l'ensemble des produits délétères résultant du processus de glycosylation non enzymatique des protéines (Koschinski T. et al., Proc Natl. Acad. Sci. USA (1997), vol.94, pp.6474-6479). Il s'agit d'un ensemble mal défini de substances parmi lesquelles on peut toutefois distinguer les composés α-cétoaldéhydes tels que le glyoxal, le méthyl-glyoxal ou le désoxyglucosone.

Une caractéristique des glycotoxines, particulièrement préjudiciable pour les organismes vivants, est leur capacité à endommager l'ADN des cellules. En effet, de telles modifications peuvent induire des mutations géniques responsables d'une instabilité génomique. A titre d'exemple, il est ainsi rapporté le caractère mutagène des sous-produits formés lorsque la protéine albumine bovine (BSA) est incubée *in vitro* avec du glucose (Ogata M. et al., J. Clin. Biochem. Nutr. (2006), vol.38, pp.176-179). Plusieurs α-cétoaldéhydes, et en particulier les glyoxal et méthyl-glyoxal susmentionnés, sont annoncés capables d'induire des mutations sur des gènes de souche bactérienne et de cellules humaines (Ueno H. et al., Mutat. Res. (1991), vol.251, pp.99-107). Plus récemment, il est rapporté le potentiel génotoxique des glycotoxines susceptible de résulter de la formation d'adduits stables par réaction avec certains nucléosides constitutifs de l'ADN (Ahmad S., Biochem. Biophys. Res. Commun. (2011), vol.15, pp.568-74). Le méthyl-glyoxal cible ainsi particulièrement les guanosines de l'ADN des chaînes nucléosidiques, entraînant alors, en l'absence d'une réparation efficace, l'apparition de mutations lors du processus de réplication de l'ADN (Wuenschell G.E. et al., Biochemistry (2010), vol.49, pp.1814-1821).

En conséquence, prenant en compte ces différents constats, la demanderesse s'est attachée à l'identification de substances, à visée cosmétique ou dermocosmétique, capables d'interférer sur le processus de glycosylation non enzymatique des protéines, avec pour objectif majeur d'éviter tout dommage affectant l'ADN des cellules cutanées et résultant de la production de glycotoxines. Il est en effet un enjeu aujourd'hui dans l'industrie cosmétique, particulièrement dans la lutte contre la sénescence prématurée des cellules cutanées, de protéger l'ADN génomique de cellules telles les kératinocytes ou les fibroblastes.

Avec cet objectif, la demanderesse a recherché plus précisément des structures originales capables de piéger, lors du processus de glycosylation non enzymatique des protéines, les sous-produits de glycosylation tels les glycotoxines génotoxiques décrites ci-avant, tout en veillant à ce que les adduits (glycotoxine-piègeur) formés ne soient pas génotoxiques, mais aussi que ces mêmes adduits ne puissent évoluer vers la formation de produits eux-mêmes génotoxiques. Il est effectivement de première importance dans le cadre d'une finalité cosmétique ou dermocosmétique de s'assurer, outre de l'innocuité des substances, également de l'innocuité des sous-produits réactionnels résultant de l'activité cosmétique visée.

Concernant l'état de la technique attaché aux objectifs de la demanderesse, l'art antérieur révèle essentiellement, à la connaissance de celle-ci, des substances ou préparations affichant un simple profil préventif ou inhibiteur dans la formation de produits de glycosylation. Ainsi, à titre d'exemple, les substances de la famille des guanidines telles l'aminoguanidine et la metformine sont d'intérêt thérapeutique pour leur capacité à se lier aux sous-produits précoces de glycosylation (Matsuki K. et al., Atherosclerosis (2009), vol.206, pp.434-8). Plusieurs sels de thiazolium, particulièrement le bromure de N-phénacyl thiazolium ou "PTB", seraient aussi d'intérêt thérapeutique en raison de leur capacité à rompre les liaisons croisées entre protéines (Ulrich P. et al., Diabetologia (1997), vol.40, pp.S157-S159).

En matière de soin cutané, certains dérivés hydroxylés de benzofuranne sont utilisés à des fins cosmétiques pour leurs propriétés limitantes, annoncées même inhibitrices, de la réaction de glycosylation non enzymatique de protéines dermiques ou kératiniques (brevet FR 2833165). Les demandeurs des brevets FR 2802425 et WO 2010/010248 sont parvenus à identifier des extraits végétaux, respectivement de la famille des Ericaceae et Sapotaceae, pour la protection de cellules et protéines cutanées avec un même résultat limitant ou inhibiteur. Mais il est à souligner que nul de ces documents n'apporte en combinaison, et un enseignement sur un bénéfice à l'encontre de l'ADN de cellules cutanées vis-à-vis spécifiquement de glycotoxines génotoxiques, et un enseignement sur le devenir des sous-produits de glycosylation détoxifiés. Plus largement, il est également cité les vertus antioxydantes de produits possédant un groupement imidazole, objets de la demande de brevet WO95/12581 déposée par la demanderesse, exploitées pour diverses applications thérapeutiques et cosmétologiques.

Avec comme base initiale de viser une structure polyamine linéaire à l'instar des substances spermine et spermidine d'intérêt contre la glycosylation non enzymatique de certaines protéines (Gugliucci A. et al. Life Sciences (2003), vol.72, pp.2603-2616), la demanderesse a ainsi découvert qu'une famille limitée de composés, obtenue par réaction de couplage entre un panel d'acides aminoalcanoiques et des éthylamines primaires portant un noyau aromatique ou un hétérocycle aromatique, présentait un comportement général d'inhibiteur des dommages de l'ADN induits par les sous-produits délétères de glycosylation tels les glycotoxines. Les composés de l'invention présentent les propriétés avantageuses suivantes :
- une capacité à protéger une protéine cible nucléaire privilégiée des glycotoxines, les histones [cf. test 1 ci-après]. Les histones constituent des protéines essentielles à l'enroulement de l'ADN autour du nucléosome, puisqu'elles orchestrent le niveau de compaction ou de relaxation de la chromatine, forme sous laquelle se présente l'ADN dans le noyau et qui permet une accessibilité des systèmes de réparation au site de dommages de l'ADN (Roberts et al., Mut. Res. (2003), vol.522, pp.45-56) ;
- une capacité à supprimer le caractère mutagène des sous-produits génotoxiques issus de la glycosylation non enzymatique d'une protéine par du fructose, reflétée par une capacité à réduire significativement, voire totalement, le nombre de mutations (dites "réverses") induites dans un procaryote de référence [cf. test 2 ci-après] ;
- une capacité à supprimer le caractère mutagène d'une glycotoxine de référence, le méthyl-glyoxal [cf. test 3 ci-après] ;
- une capacité à interférer sur le processus de glycosylation non enzymatique avec la propriété de ne pas former, en conditions glycosylantes, de sous-produits génotoxiques, ou de ne pas évoluer dans ces conditions vers la formation de tels sous-produits [cf. test 4 ci-après].

L'invention a donc pour premier objet une famille de composés stables aryléthylamides polyaminés, caractérisée en ce que ladite famille est représentée par la formule générale (II) suivante : 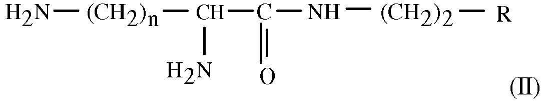 dans laquelle : n = 1 à 2
et R=

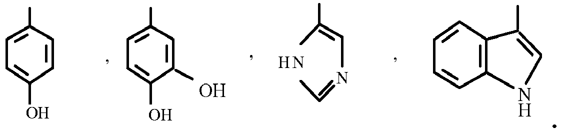

Suivant un mode de réalisation préféré de l'invention, les composés de l'invention sont représentés par la formule (III) ci-après : 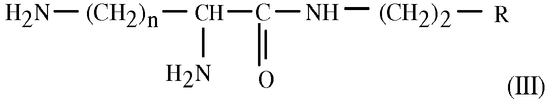 dans laquelle : n = 1 à 2
et R =

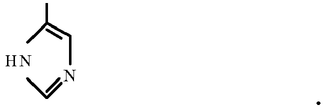

L'invention a également pour objet les sels pharmaceutiquement acceptables des composés de formule (II) ou (III).

A titre d'exemples non limitatifs de composés aryléthylamides polyaminés de formule (II), on peut citer notamment les composés suivants :
- *N*-((4-imidazolyl)éthyl)-α,γ-diaminobutanamide
- *N*-((4-imidazolyl)éthyl)-α,ß-diaminopropanamide
- *N*-((3-indolyl)éthyl)-α,ß-diaminopropanamide
- *N*-((4-hydroxyphényl)éthyl)-α,ß-diaminopropanamide
- *N*-((3,4-dihydroxyphényl)éthyl)-α,ß-diaminopropanamide.

Les composés aryléthylamides polyaminés de formule (III) sont les suivants :
- *N*-((4-imidazolyl)éthyl)-α,γ-diaminobutanamide
- *N*-((4-imidazolyl)éthyl)-α,ß-diaminopropanamide.

Suivant un mode de réalisation encore plus avantageux de l'invention, les formules (II) et (III) susmentionnées visent tout particulièrement les composés aryléthylamides polyaminés *N*-((4-imidazolyl)éthyl)-α,γ-diaminobutanamide et N-((4-imidazolyl)éthyl)-α,ß-diaminopropanamide, plus particulièrement le composé *N*-((4-imidazolyl)éthyl)-α,β-diaminopropanamide.

Les composés de l'invention peuvent être synthétisés sous forme de base libre ou de sel par des méthodes bien connues de l'homme du métier, par exemple en suivant une approche chimique classique pour la synthèse de peptides (séquences de protection, couplage et déprotection). Les sels des composés de l'invention peuvent être en particulier obtenus par réaction desdits composés avec un acide inorganique ou un acide organique.

Selon un deuxième aspect, l'invention s'étend également à une composition, à usage cosmétique ou dermocosmétique, destinée à prévenir ou limiter le "risque génotoxique" associé au processus de glycosylation non enzymatique, et en particulier associé à la formation de sous-produits de glycosylation génotoxiques (glycotoxines). Ladite composition comprend en association avec tout adjuvant physiologiquement compatible avec la peau, à titre d'ingrédient actif principal, un composé de formule générale (II) ou (III) tel que défini ci-avant, se présentant sous forme solide pulvérulente, soluble en milieu aqueux ou hydroalcoolique mais insoluble dans l'éthanol, stable sur une large gamme de pH (3-9).

Dans le cadre de la présente invention, on entend par "ingrédient actif principal" une substance active capable de de détoxifier les glycotoxines et de s'opposer à leur formation et leurs effets délétères.

Avantageusement, la quantité de composé de formule générale (II) ou (III) dans la composition ci-dessus est comprise entre 0,01 % et 1 % en poids par rapport au poids total de la composition, de préférence entre 0,01 % et 0,5 % en poids, plus particulièrement encore entre 0,02 % et 0,2 % en poids.

Les compositions selon l'invention sont adaptées à une administration par voie topique cutanée, présentées sous toutes les formes normalement utilisées pour une telle administration. A titre indicatif et non limitatif, les compositions peuvent se présenter sous la forme de suspensions, lotions, crèmes, gels aqueux ou hydroalcooliques, poudres et émulsions multiples pouvant être éventuellement des microémulsions ou des nanoémulsions, etc.

Les compositions selon l'invention peuvent aussi être formulées pour une administration par voie orale illustrée de façon non limitative par un comprimé, un gélule, une capsule, un cachet, un sachet, une pâte, un liquide (émulsionné ou non) etc.

Les compositions selon l'invention peuvent contenir comme adjuvant physiologiquement acceptable au moins un adjuvant connu de l'Homme du métier et acceptable dans les domaines cosmétique ou dermocosmétique, choisi parmi les huiles, les cires, les élastomères de silicone, les tensioactifs, les co-tensioactifs, les épaississants et/ou gélifiants, les humectants, les émollients, les filtres solaires organiques ou inorganiques, les photostabilisants, les conservateurs à l'exception des conservateurs donneurs d'aldéhydes, les colorants, les agents matifiants, les agents tenseurs, les séquestrants, les parfums, etc., et leurs mélanges.

Les compositions selon l'invention peuvent en outre comprendre un ou plusieurs actifs additionnels, l'Homme du métier veillant toutefois à ce que les éventuels composés complémentaires actifs, ainsi que leurs proportions, soient choisis de telle manière à ce que les propriétés avantageuses reconnues aux compositions selon l'invention ne soient pas altérées. Ces actifs additionnels peuvent être choisis, sans que cette liste ne soit limitative, parmi les agents déglycants, les agents stimulant la synthèse de collagène ou d'élastine ou prévenant leur dégradation, les agents stimulant la synthèse de glycosaminoglycanes ou de protéoglycanes ou prévenant leur dégradation, les agents augmentant la prolifération cellulaire, les agents dépigmentants ou pro-pigmentants, les agents antioxydants ou anti-radicalaires ou anti-pollution, les agents hydratants, les agents stimulant la lipolyse, les agents drainants ou détoxifiants, les agents anti-inflammatoires, les agents accélérateurs de pénétration, les agents desquamants, les agents apaisants et/ou anti-irritants, les agents astringents, les agents agissant sur la microcirculation, etc., et leurs mélanges.

Les compositions selon l'invention visent à prévenir ou lutter contre tout désordre cutané associé au processus de glycosylation non enzymatique des protéines et à la formation de glycotoxines génotoxiques, choisi parmi la sénescence prématurée des cellules cutanées, l'éclaircissement de la peau, etc. Dans de telles compositions, le composé aryléthylamide polyaminé est préférentiellement de formule (III), plus particulièrement encore le composé N-((4-imidazolyl)éthyl)-α,ß-diaminopropanamide.

Un autre objet de l'invention concerne l'utilisation cosmétique ou dermocosmétique d'un composé aryléthylamide polyaminé comme agent inhibiteur des dommages de l'ADN induits par les glycotoxines génotoxiques, ledit composé aryléthylamide polyaminé étant de formule générale (II) suivante : 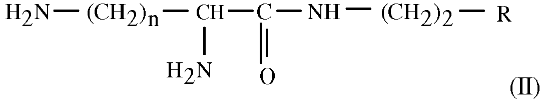 dans laquelle : n = 1 à 2
et R =

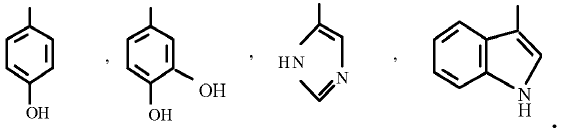

De préférence dans l'utilisation ci-dessus, il est sélectionné un composé de formule (III) suivante : 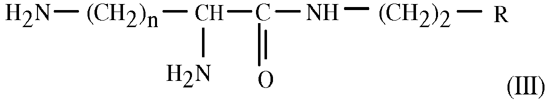 dans laquelle : n = 1 à 2
et R= 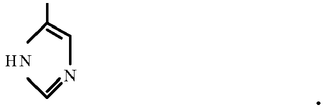 et de manière préférée le composé *N*-((4-imidazolyl)éthyl)-α,β-diaminopropanamide.

Un autre objet de l'invention concerne une méthode de soin cosmétique qui comprend l'application sur au moins une partie de la peau du corps, d'une composition cosmétique telle que défini ci-dessus en quantité efficace pour lutter contre les désordres cutanés associés aux sous-produits de la glycosylation non enzymatique des tissus cutanés.

### Exemple 1 :

A titre d'illustration, il est mentionné ci-après deux exemples de formulation de composition selon l'invention, contenant un composé aryléthylamide polyaminé de formule générale (II) précitée :
Formule A (crème)

| | |
|---|---|
| *N*-((4-imidazolyl)éthyl)-α,ß-diaminopropanamide | 0,05 % |
| Polyisobutène hydrogéné | 7 % |
| Myristate d'isobutyle | 3 % |
| Palmitate de cétyle | 7 % |
| Monostéarate d'éthylène glycol | 5 % |
| Laurate sorbitan | 2 % |
| Polysorbate 20 | 2 % |
| Carbomer (copolymère d'acrylate/acrylamide & huile minérale) | 0,3 % |
| Phénoxyéthanol | 0,5 % |
| Eau | qsp 100% |

Formule B (gel)

| | |
|---|---|
| *N*-((4-imidazolyl)éthyl)-α,γ-diaminobutanamide | 0,1 % |
| Carbomer (copolymère d'acrylate/acrylamide & huile minérale) | 1,5 % |
| Benzoate de sodium | 0,2 % |
| Acide sorbique | 1 % |
| 1,3-butanediol | 10 % |
| Glycérine | 5 % |
| Soude | 0,13 % |
| Phénoxyéthanol | 0,9 % |
| Eau | qsp 100% |

### Exemple 2 :

L'invention est illustrée ci-après, à titre purement indicatif, par les tests suivants évoqués plus haut dans la description de l'invention (tests 1 à 4).

Il est aussi à signaler que les premiers résultats d'études in vivo menées sur l'Homme (Test d'applications répétées sous patch, prestataire : Sté Evic Romania) indiquent une bonne tolérance cutanée d'un composé aryléthylamide polyaminé selon la présente invention.

### Test 1 : mise en évidence de la capacité des composés aryléthylamides polyaminés de formule générale (II) à protéger des protéines essentielles à l'enroulement de l'ADN nucléaire, les histones, vis-à-vis d'une glycotoxine de référence, le méthvl-glyoxal (MGO)

Une solution d'histones purifiées (5mg/ml), enrichie en histone H1, est incubée en présence de la glycotoxine de référence MGO (10mM) durant 24h à 37°C sous agitation. Afin de suivre spécifiquement la fluorescence des produits issus de la glycosylation non enzymatique des histones, il est nécessaire de s'affranchir de la fluorescence du MGO, ainsi que de celle des produits glycotoxines-piégeur, pour cela le mélange de réaction est dialysé. Le mélange est introduit dans une cassette de dialyse et subira 3 bains successifs de 3 heures chacun dans une solution de tampon NaH₂PO₄ (83,3mM), dans lequel est dilué le MGO. La fluorescence du mélange dialysé, indicative de l'endommagement des histones par réaction avec le MGO (formation d'adduits) est mesurée par fluorimétrie (À d'excitation : 340nm ; λ d'émission : 470nm). L'unité de mesure correspond à des RFU (de l'anglais Relative Fluorescence Unit).

**Tableau 1**

| **Composé** | **RFU** |
|---|---|
| histones + MGO 10 mM (contrôle) | 62231 |
| *N*-((4-imidazolyl)éthyl)-α,ß-diaminopropanamide 10 mM + histones + MGO 10 mM | 20441 |
| *N*-((4-imidazolyl)éthyl)-α,γ-diaminobutanamide 10 mM + histones + MGO 10 mM | 21526 |

Les résultats soulignent, pour les composés *N*-((4-imidazolyl)éthyl)-α,ß-diaminopropanamide et N-((4-imidazolyl)éthyl)-α,γ-diaminobutanamide selon l'invention, une capacité à diminuer fortement la formation de produits de glycosylation non-enzymatique générés par réaction de la glycotoxine avec les histones.

### Test 2 mise en évidence de la capacité des composés aryléthylamides polyaminés de formule générale (II) à détoxifier les glycotoxines libérées par une protéine glycosylée

L'étude a été réalisée sur une souche bactérienne *Salmonella typhimurium* (TA 100) appartenant aux souches de référence utilisées pour le test réglementaire d'Ames (ICH steering committee, 19 Juillet 1995, Guidance on specific aspect of regulatory genotoxicity tests for pharmaceuticals), l'objectif étant de déterminer dans les colonies de souches mutées le nombre de révertants, indicateurs de la mutagénèse, du système caséine-fructose, en présence ou en absence d'un composé selon l'invention.

Expérimentalement, 2,7g de D-(+)-fructose sont solubilisés à température ambiante dans 100mL de tampon phosphate. Après prélèvement de 20g de la solution limpide incolore (C_{fructose} = 150mM), 0,600g de caséinate de sodium sont introduits jusqu'à solubilisation complète. La solution est alors introduite en 2 x 10mL dans des tubes à essai à bouchon vissé, puis placée dans un bain d'huile à 120°C pendant 1h.

Parallèlement, la souche TA 100 est mise en culture dans du Nutrient Broth Oxoid N°2 (NBO2) supplémentée en Ampicilline. La culture est placée une nuit à 37°C dans un agitateur orbital (88 tr/min) de façon à ce que les bactéries se trouvent en phase exponentielle de croissance (10⁷ à 10⁹ bactéries/ml). Par ailleurs, 0,92g de composé selon l'invention, le *N*-((4-imidazolyl)éthyl)-α,ß-diaminopropanamide sont solubilisés dans une solution tampon de phosphate salin (PBS) fraîchement préparée. A différentes concentrations (400 µl) de composé selon l'invention sont ajoutés successivement 500 µl de caséine glyquée susmentionnée et 100 µl de la suspension bactérienne susmentionnée, le mélange résultant étant incubé 30 minutes à 37°C sous agitation orbitale (122 tr/min). 2 ml de gélose molle supplémentée en Histidine Biotine et maintenue à 45°C sont ajoutés et, après mélange au vortex, sont déposés sur boîte de pétri contenant de la gélose dure Vogel-Bonner. Après solidification les boites ont été transférées dans l'incubateur à 37°C pendant 48-72h.

L'essai a été réalisé en absence d'activateur métabolique selon la méthode de pré-incubation sur la souche TA 100. Le comptage des colonies est alors réalisé manuellement.

Les résultats sont présentés dans le tableau 2 ci-après, en comparaison à un témoin négatif (tampon phosphate PBS).

**Tableau 2**

| **Composé** | **Nbre de révertants** | **R** |
|---|---|---|
| témoin (PBS) | 84 | 1 |
| caséine fructosylée | 138 | 1,64 |
| *N*-((4-imidazolyl)éthyl)-α,ß-diaminopropanamide (7,5 mM) | 119 | 1,42 |
| *N*-((4-imidazolyl)éthyl)-α,ß-diaminopropanamide (15 mM) | 93 | 1,11 |

Les résultats soulignent que le composé *N*-((4-imidazolyl)éthyl)-α,ß-diaminopropanamide selon l'invention est capable de diminuer fortement la toxicité des glycotoxines formées. Un effet dose est en outre observé.

### Test 3 : mise en évidence de la capacité des composés aryléthylamides polyaminés de formule générale (II) à détoxifier une glycotoxine de référence, le méthyl-glyoxal (MGO)

L'étude a été réalisée de manière quasi-identique au test 2 ci-avant, avec toutefois une solution de MGO à la concentration de 140 µM venant remplacer les 500 µl de caséine glyquée. Outre le composé *N*-((4-imidazolyl)éthyl)-α,β-diaminopropanamide, on a également testé les composés suivants selon l'invention, à différentes concentrations :
- *N*-((4-hydroxyphényl)éthyl)-α,ß-diaminopropanamide
- *N*-((4-imidazolyl)éthyl)-α,γ-diaminobutanamide
- *N*-((3,4-dihydroxyphényl)éthyl)-α,ß-diaminopropanamide.
Les résultats sont rassemblés dans le tableau 3 ci-après, en comparaison au tampon phosphate PBS (témoin négatif).

**Tableau 3**

| **Composé** | **Nbre de révertants** |
|---|---|
| Témoin (PBS) | 72 |
| Méthyl-glyoxal 280 µM | 308 |
| *N*-((4-imidazolyl)éthyl)-α,ß-diaminopropanamide (75 µM) | 141 |
| *N*-((4-imidazolyl)éthyl)-α,ß-diaminopropanamide (750 µM) | 95 |
| | |

| **Composé** | **Nbre de révertants** |
|---|---|
| Témoin (PBS) | 91 |
| Méthyl-glyoxal 140 µM | 161 |
| *N*-((4-hydroxyphényl)éthyl)-α,ß-diaminopropanamide (75 µM) | 152 |
| | |

| **Composé** | **Nbre de révertants** |
|---|---|
| Témoin (PBS) | 91 |
| Méthyl-glyoxal 140 µM | 161 |
| *N*-((4-imidazolyl)éthyl)-α,γ-diaminobutanamide (75 µM) | 154 |
| *N*-((4-imidazolyl)éthyl)-α,γ-diaminobutanamide (750 µM) | 116 |
| | |

| **Composé** | **Nbre de révertants** |
|---|---|
| Témoin (PBS) | 91 |
| Méthyl-glyoxal 140 µM | 161 |
| *N*-((3,4-dihydroxyphényl)éthyl)-α,ß-diaminopropanamide (75 µM) | 161 |
| *N*-((3,4-dihydroxyphényl)éthyl)-α,ß-diaminopropanamide (750 µM) | 111 |

Les résultats soulignent qu'un panel assez vaste de composés selon l'invention est capable de réduire fortement la mutagénicité du méthyl-gloxal.

### Test 4 mise en évidence de la capacité des composés aryléthylamides polyaminés de formule générale (II) à interférer sur le processus de glycosylation non enzymatique sans former de sous-produits mutagènes

L'étude a été réalisée de manière quasi-identique au test 2 ci-avant, avec toutefois du D-glucose venant remplacer le D-(+)-fructose et selon les conditions suivantes. Expérimentalement, 18,02g de D-glucose sont solubilisés à température ambiante dans 100mL d'eau. Après prélèvement de 10mL de la solution, 10 mM de composé selon l'invention, le N-((4-imidazolyl)éthyl)-α,ß-diaminopropanamide, sont introduits avec ajustement du pH par addition de lessive de soude. Le mélange réactionnel résultant est alors introduit dans un tube à essai à bouchon vissé, puis chauffé 80 minutes à 100°C.

Parallèlement, la souche TA 100 est mise en culture dans du Nutrient Broth Oxoid N°2 (NBO2) supplémentée en Ampicilline. La culture est placée une nuit à 37°C dans un agitateur orbital (88 tr/min) de façon à ce que les bactéries se trouvent en phase exponentielle de croissance (10⁷ à 10⁹ bactéries/ml).

A 100 µl de solution d'essai sont ajoutées 100 µl de la suspension bactérienne susmentionnée et 500 µl de tampon phosphate PBS, le mélange résultant étant incubé 1h à 37°C sous agitation orbitale (122 tr/min). 2 ml de gélose molle supplémentée en Histidine Biotine et maintenue à 45°C sont ajoutés et, après mélange au vortex, sont déposés sur boîte de pétri contenant de la gélose dure Vogel-Bonner. Après solidification les boites ont été transférées dans l'incubateur à 37°C pendant 48-72h.

L'essai a été réalisé en absence d'activateur métabolique selon la méthode de pré-incubation sur la souche TA 100. Le comptage des colonies est alors réalisé manuellement. Les résultats sont présentés dans le tableau 4 ci-après, en comparaison à deux témoins : une référence négative (tampon phosphate PBS) et une référence positive mutagène (azoture de sodium NaN₃).

**Tableau 4**

| **Composé** | **Nbre de révertants** |
|---|---|
| Témoin (PBS) (révertants spontanés) | 88 |
| Témoin (NaN₃) (référence positive) | 211 |
| *N*-((4-imidazolyl)éthyl)-α,ß-diaminopropanamide (1,667 µl/boîte) | 104 |
| *N*-((4-imidazolyl)éthyl)-α,ß-diaminopropanamide (25 µl/boîte) | 95 |
| *N*-((4-imidazolyl)éthyl)-α,ß-diaminopropanamide (50 µl/boîte) | 90 |
| *N*-((4-hydroxyphényl)éthyl)-α,ß-diaminopropanamide (50 µl/boîte) | 93 |
| *N*-((4-imidazolyl)éthyl)-α,γ-diaminobutanamide (50 µl/boîte) | 93 |

Les résultats soulignent que les composés aryléthylamides polyaminés de formulé générale (II) selon l'invention ne génèrent pas, malgré les conditions glycosylantes, de sous-produits mutagènes.

## Revendications

1. Composé aryléthylamide polyaminé, caractérisé en ce ledit composé est représenté par la formule générale (II) suivante : 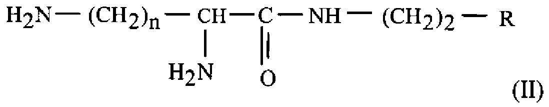 dans laquelle : n = 1 à 2
et R=

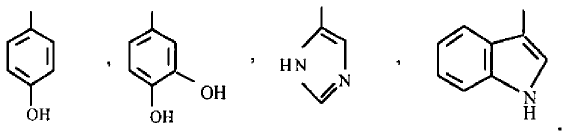

2. Composé aryléthylamide polyaminé selon la revendication 1, **caractérisé en ce qu'**il s'agit du *N*-((4-imidazolyl)éthyl)-α,γ-diaminobutanamide ou du *N*-((4-imidazolyl)éthyl)-α,β-diaminopropanamide.

3. Composé aryléthylamide polyaminé selon la revendication 2, **caractérisé en ce qu'**il s'agit du *N*-((4-imidazolyl)éthyl)-α,β-diaminopropanamide.

4. Composition cosmétique ou dermocosmétique, **caractérisée en ce qu'**elle comprend, en association avec tout adjuvant physiologiquement compatible avec la peau, à titre d'ingrédient actif principal, un composé tel que défini dans l'une quelconque des revendications 1 à 3.

5. Composition selon la revendication 4, **caractérisée en ce que** ledit composé est choisi parmi le *N*-((4-imidazolyl)éthyl)-α,γ-diaminobutanamide et le *N*-((4-imidazolyl)éthyl)-α,ß-diaminopropanamide.

6. Composition selon la revendication 5, **caractérisée en ce que** ledit composé est le *N*-((4-imidazolyl)éthyl)-α,β-diaminopropanamide.

7. Composition selon l'une quelconque des revendications 4 à 6, **caractérisée en ce que** la quantité dudit composé est comprise entre 0,01 % et 1 % en poids par rapport au poids total de la composition.

8. Composition selon l'une quelconque des revendications 4 à 7, **caractérisée en ce qu'**elle comprend en outre un ou plusieurs actifs choisis parmi les agents déglycants, les agents stimulant la synthèse de collagène ou d'élastine ou prévenant leur dégradation, les agents stimulant la synthèse de glycosaminoglycanes ou de protéoglycanes ou prévenant leur dégradation, les agents augmentant la prolifération cellulaire, les agents dépigmentants ou pro-pigmentants, les agents antioxydants ou anti-radicalaires ou anti-pollution, les agents hydratants, les agents stimulant la lipolyse, les agents drainants ou détoxifiants, les agents anti-inflammatoires, les agents accélérateurs de pénétration, les agents desquamants, les agents apaisants et/ou anti-irritants, les agents astringents, les agents agissant sur la microcirculation, et leurs mélanges.

9. Composé de formule générale (II) : 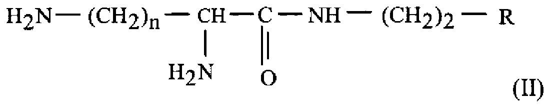 dans laquelle : n = 1 à 2
et R = 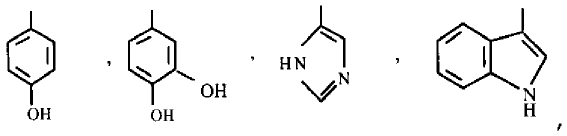 pour son utilisation pour prévenir ou lutter contre les désordres cutanés associés à la formation de sous-produits de glycosylation et de glycotoxines génotoxiques.

10. Composé pour l'utilisation selon la revendication 9, **caractérisé en ce que** ledit désordre cutané est choisi parmi la sénescence prématurée des cellules cutanées et l'éclaircissement de la peau.

11. Composé pour l'utilisation selon la revendication 9 ou la revendication 10, **caractérisée en ce que** ledit composé est tel que défini dans la revendication 2 ou la revendication 3.

## Patentansprüche

1. Polyamin-Arylethylamid-Verbindung, **dadurch gekennzeichnet, dass** die Verbindung durch die folgende allgemeine Formel (II) repräsentiert ist: 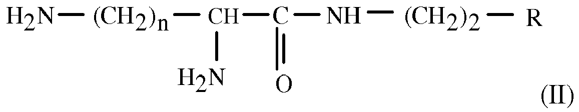 worin: n = 1 bis 2
und R =

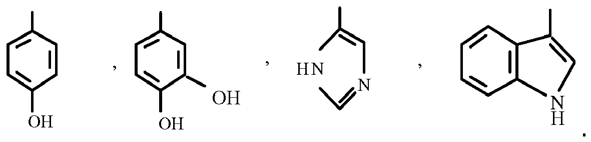

2. Polyamin-Arylethylamid-Verbindung nach Anspruch 1, **dadurch gekennzeichnet, dass** es sich um *N*-((4-imidazolyl)ethyl)-α,γ-diaminobutanamid oder um *N*-((4-imidazolyl)ethyl)-α,ß-diaminopropanamid handelt.

3. Polyamin-Arylethylamid-Verbindung nach Anspruch 2, **dadurch gekennzeichnet, dass** es sich um *N*-((4-imidazolyl)ethyl)-α,ß-diaminopropanamid handelt.

4. Kosmetische oder dermokosmetische Zusammensetzung, **dadurch gekennzeichnet, dass** sie, in Verbindung mit jedwedem physiologisch hautverträglichen Adjuvans, als Hauptwirkbestandteil eine Verbindung, wie sie in einem der Ansprüche 1 bis 3 definiert ist, umfasst.

5. Zusammensetzung nach Anspruch 4, **dadurch gekennzeichnet, dass** die Verbindung aus *N*-((4-imidazolyl)ethyl)-α,γ-diaminobutanamid und *N*-((4-imidazolyl)ethyl)-α,ß-diaminopropanamid ausgewählt ist.

6. Zusammensetzung nach Anspruch 5, **dadurch gekennzeichnet, dass** die Verbindung *N*-((4-imidazolyl)ethyl)-α,ß-diaminopropanamid ist.

7. Zusammensetzung nach einem der Ansprüche 4 bis 6, **dadurch gekennzeichnet, dass** die Menge der Verbindung zwischen 0,01 und 1 Gew.-% bezogen auf das Gesamtgewicht der Zusammensetzung liegt.

8. Zusammensetzung nach einem der Ansprüche 4 bis 7, **dadurch gekennzeichnet, dass** sie ferner einen oder mehrere Wirkstoffe umfasst, die aus den Deglykationsmitteln, den Mitteln, die die Synthese von Kollagen oder Elastin stimulieren oder deren Abbau vorbeugen, den Mitteln, die die Synthese von Glykosaminoglykanen oder Proteoglykanen stimulieren oder deren Abbau vorbeugen, den die Zelleproliferation erhöhenden Mitteln, den depigmentierenden oder propigmentierenden Mitteln, den Antioxidantien oder den Radikalfangendern oder den schadstoffhemmenden Mitteln, den feuchtigkeitsspendenden Mitteln, den Lipolysestimulierenden Mitteln, den entwässernden oder entgiftenden Mitteln, den entzündungshemmenden Mitteln, den Penetrationsbeschleunigern, den abschuppenden Mitteln, den beruhigenden und/oder irritationshemmenden Mitteln, den adstringierenden Mitteln, den auf die Mikrozirkulation wirkenden Mitteln sowie ihren Mischungen ausgewählt sind.

9. Verbindung der allgemeinen Formel (II): 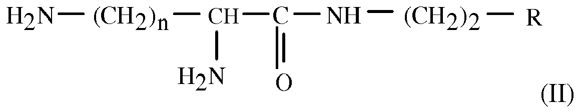 worin: n = 1 bis 2
und R= 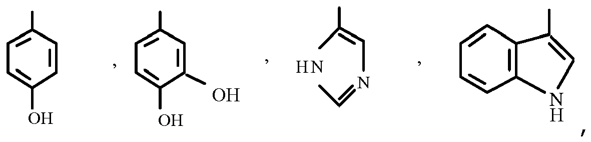 für ihre Verwendung zum Vorbeugen oder Bekämpfen von Hautstörungen, die mit der Bildung von Nebenprodukten einer Glykosylierung und von genotoxischen Glykotoxinen verbunden sind.

10. Verbindung für die Verwendung nach Anspruch 9, **dadurch gekennzeichnet, dass** die Hautstörung aus der vorzeitigen Seneszenz der Hautzellen und der Aufhellung der Haut ausgewählt ist.

11. Verbindung für die Verwendung nach Anspruch 9 oder Anspruch 10, **dadurch gekennzeichnet, dass** die Verbindung so ist wie in Anspruch 2 oder Anspruch 3 definiert.

## Claims

1. A polyamine arylethylamide compound, wherein said compound is represented by the following general formula (II): 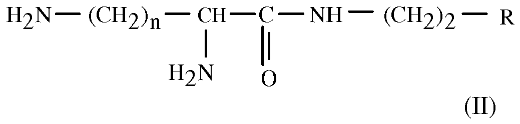 wherein : n = 1 to 2
and R =

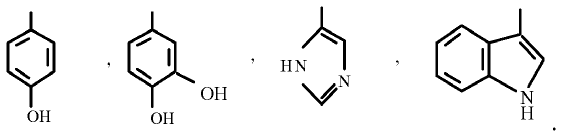

2. The polyamine arylethylamide compound according to claim 1, which is N-((4-imidazolyl)ethyl)-α,γ-diaminobutanamide, or *N*-((4-imidazolyl)ethyl)-α,β-diamino-propanamide.

3. The polyamine arylethylamide compound according to claim 2, which is N-((4-imidazolyl)ethyl)-α,β-diaminopropanamide.

4. A cosmetic or dermocosmetic composition, which comprises as main active ingredient a compound as defined in any one of claims 1 to 3, in combination with an additive which is physiologically compatible with skin.

5. The composition according to claim 4, wherein said compound is N-((4-imidazolyl)ethyl)-α,γ-diaminobutanamide or *N*-(((4-imidazolyl)-ethyl)-α,β-diaminopropanamide.

6. The composition according to claim 5, wherein said compound is N-((4-imidazolyl)ethyl)-α,β-diaminopropanamide.

7. The composition according to any one of claims 4 to 6, wherein the amount of said compound is comprised between 0.01 % and 1 % by weight based on the total weight of the composition.

8. The composition according to any one of claims 4 to 7, which further comprises one or several active ingredients selected from deglycation agents, agents that stimulate the synthesis of collagen or elastin or prevent their degradation, agents that stimulate the synthesis of glycosaminoglycans or proteoglycans or prevent their degradation, agents that increase cell proliferation, depigmenting or pro-pigmenting agents, antioxidant or anti-radical or anti-pollution agents, moisturizing agents, agents that stimulate lipolysis, draining or detoxifying agents, anti-inflammatory agents, penetration enhancer agents, desquamative agents, soothing or anti-irritating agents, astringent agents, agents that act on the microcirculation, and their mixtures.

9. A compound of general formula (II): 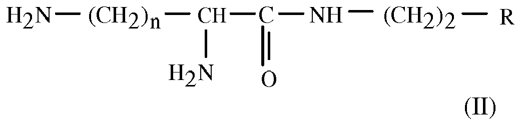 wherein : n = 1 to 2
and R = 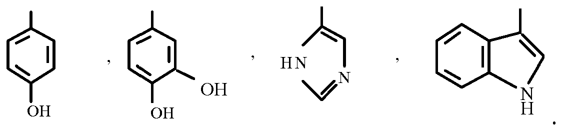 for use in the prevention or treatment of skin disorders associated with the formation of glycosylation by-products or with the formation of genotoxic glycotoxins.

10. The compound for the use according claim 9, wherein said skin disorder is premature senescence of skin cells, or skin lightening.

11. The compound for the use according to claim 9 or claim 10, wherein said compound is as defined in claim 2 or claim 3.
